# EUROPEAN PATENT SPECIFICATION

(11) **EP 1 501 774 B1**
(45) Date of publication and mention of the grant of the patent: **12.08.2009**
(21) Application number: 03749867.2
(22) Date of filing: 06.05.2003
(51) Int. Cl.: C07C 33/02, C07C 69/24, C07C 47/21, A61K 31/22, A61K 31/11, A61K 31/045, A61P 29/00, A61P 35/00, A61P 39/06

(54) **POLYUNSATURATED LINEAR ALDEHYDES AND THEIR DERIVATIVES WITH ANTI-RADICAL ACTIVITY**
MEHRFACH UNGESÄTTIGTE, GERADKETTIGE ALDEHYDE UND DEREN DERIVATE MIT ANTI-RADIKALWIRKUNG
ALDEHYDES LINEAIRES POLYINSATURES ET LEURS DERIVES A ACTIVITE ANTI-RADICALAIRE

(30) Priority: 07.05.2002 IT MI20020960
(43) Date of publication of application: 02.02.2005
(73) Proprietor: Giuliani S.p.A., 20129 Milano (IT)
(72) Inventor: STRADI, Riccardo, I-20133 Milano (IT); BERTELLI, Aldo, I-20122 Milano (IT); PINI, Elena, I-20133 Milano (IT)
(74) Representative: Bianchetti, Giuseppe
(86) International application number: PCT/EP2003/004720
(87) International publication number: WO 2003/095403

(56) References cited:
- US-A- 3 679 792
- DATABASE BIOSIS [Online] BIOSCIENCES INFORMATION SERVICE, PHILADELPHIA, PA, US; 13 January 2003 (2003-01-13) LEE PING C ET AL: "Short term feeding of hexadienal to postnatal rats: Effects on stomach aldehyde dehydrogenase." Database accession no. PREV200300137234 XP002248781 & TOXICOLOGY LETTERS (SHANNON), vol. 136, no. 3, 13 January 2003 (2003-01-13), pages 173-181, ISSN: 0378-4274
- DATABASE BIOSIS [Online] BIOSCIENCES INFORMATION SERVICE, PHILADELPHIA, PA, US; December 2001 (2001-12) NYSKA ABRAHAM ET AL: "Glutathione S-transferase Pi expression in forestomach carcinogenesis process induced by gavage-administered 2,4-hexadienal in the F344 rat." Database accession no. PREV200200138746 XP002248782 & ARCHIVES OF TOXICOLOGY, vol. 75, no. 10, December 2001 (2001-12), pages 618-624, December, 2001 ISSN: 0340-5761

## Description

This invention relates to polyunsaturated linear aldehydes and their derivatives with anti-radical activity.

More particularly, this invention relates to the use of polyunsaturated linear aldehydes which can be extracted from parrot feathers and tissues or prepared synthetically, and of derivatives of said aldehydes such as fatty esters of alcohols obtained by reduction of the aldehyde group, or derivatives of inclusion of said aldehydes in cyclodextrins, as antioxidant and anti-inflammatory agents.

This invention also relates to novel derivatives of said aldehydes, such as some alcohols obtained by reduction of the aldehyde group, fatty esters of alcohols and derivatives of inclusion of aldehydes in cyclodextrins. Said polyunsaturated aldehydes and the derivatives thereof will hereafter be generically referred to as "parrodienes".

It has been found that the parrodienes of the invention possess antioxidant and anti-inflammatory activity and are therefore useful in preventing the damage caused by free radicals, and in particular in the prevention and treatment of, and the prevention of alterations caused by skin ageing.

Numerous natural products of plant origin, present in the diets of various populations, possess a preventive or curative activity against numerous organic disorders and alterations characteristic of various diseases.

These therapeutic properties are generally indicated by folk tradition, and their validity has been investigated by modern chemical, biological, pharmacological and clinical techniques.

Far fewer natural products of animal origin included in the diet have been found to have therapeutic or preventive properties against particular disorders or diseases.

Known examples are polyunsaturated fatty acids extracted from fish (1, 2, 3), glycosaminoglycans extracted from the cartilage of animals such as the shark (3, 4, 5, 6, 8), glycoproteins such as lactoferrin extracted from milk or colostrum (9, 10), and lipid extracts of molluscs such as oysters (11, 12).

However, the biological activity of the polyunsaturated linear aldehydes present in the feathers and tissues of parrots is wholly unexplored.

Folk traditions of Brazilian and Venezuelan people hand down that external application of the feathers of these birds heals skin infections, burns and poisonous insect bites, and that eating their meat cures intestinal disorders, infections and tumours. It is known that the coloured plumage of birds have numerous types of carotenoids which are absorbed through the diet and can bond directly with the keratin in the feathers or undergo a complex metabolic transformation.

Lutein, zeaxanthin and beta-cryptoxanthins are carotenoids frequently found in foods (berries, fruit, seeds, flowers and insects), which can be absorbed by tissues and feathers with no metabolic modifications to their structure, whereas other compounds, such as picofulvins, are the result of molecular modification (13, 14, 15, 16). The psittacofulvins or the mixture of parrodienes (also indicated here by the name of Parrodin) are the result of metabolic changes typical of various species of parrot (*Poicephalus rufiventris, Ara macao, Ara manilata, Ara ararauna, Psittacus erithacus, Aratinga canicularis, Aratinga acuticaudata, Psittacula krameri*, etc.).

The parrodienes according to the invention have an antioxidant activity and in general a protective effect against the free radicals, which may explain the exceptional longevity of these animals (they can live for up to 100 years), their learning ability, and above all their lack of diseases, especially tumours.

The compounds of the general formula (I) 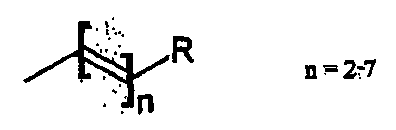 wherein
n = 2 - 7,
R = CHO, CH₂OH, CH₂O-CO-R', wherein -CO-R' is the residue of a fatty acid with 12-22 carbon atoms,
were first obtained (R = CHO) as polyene aldehydes, the synthesis of which was described for the first time by Kuhn in 1937 (17, 18). Aldehydes with an odd number of conjugated double bonds were obtained by autocondensation of crotonaldehyde in the presence of the catalyst piperidinium acetate (scheme 1A). Aldehydes with an even number of double bonds were obtained by condensation between crotonaldehyde and acetaldehyde, again in the presence of the catalyst piperidinium acetate (scheme 1B).

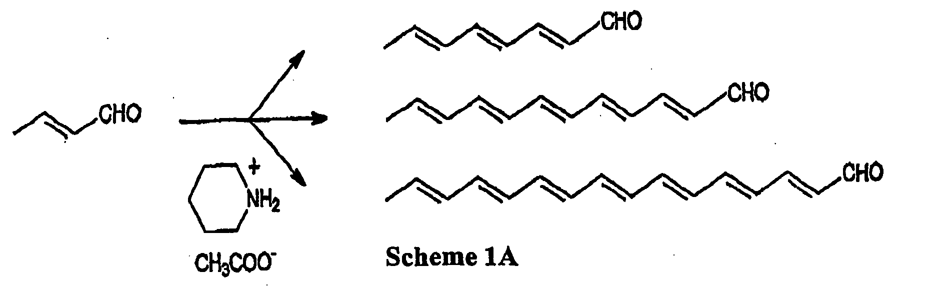

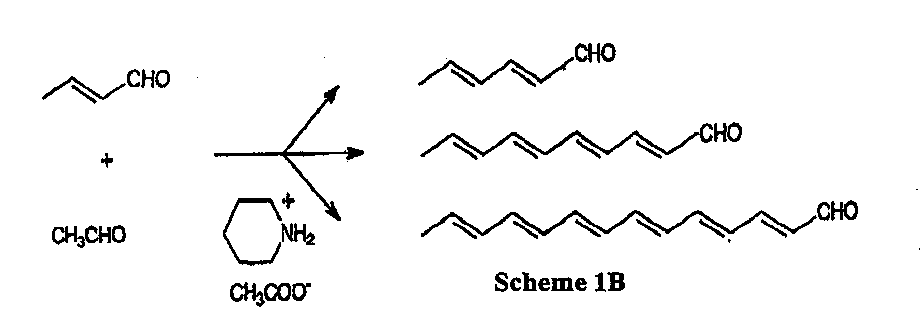

The corresponding alcohols (R = CH₂OH) were obtained by reduction of the aldehydes with NaBH₄; the esters were obtained by esterification with the required acyl halides, especially chlorides.

Examples of preparation of the compounds according to the invention are set out below.

### Example 1A - Preparation of aldehydes with an odd number of double bonds

Formula (**I**),n = 3: 2,4,6-octatrienal
n = 5: 2,4,6,8,10-dodecapentaenal
n = 7: 2,4,6,8,10,12,14-hexadecaheptaenal

250 ml of crotonaldehyde are added to a 1-litre flask and keep under magnetic stirring for 15 minutes under nitrogen flow. 2.5 ml of piperidine and 2.5 ml of acetic acid are slowly dropped therein (the reaction is exothermic) and the mixture is left under magnetic stirring in a nitrogen atmosphere at 50°C for 30 minutes.

The reaction mixture is cooled in an ice bath and added with 600 ml of ethyl ether, still under magnetic stirring. The brown precipitate formed is filtered and recrystallized from toluene: 120 mg of 2,4,6,8,10,12,14-hexadecaheptaenal is obtained.

The red ether solution is extracted with 200 x 5 ml of distilled water to wash away the unreacted surplus crotonaldehyde; the organic phase is dried over sodium sulphate and evaporated under pressure (30 mmHg). The brownish-red residue is taken up with 30 ml of 80% methanol and left in the refrigerator overnight at 3°C. 2,4,6,8,10-dodecapentaenal is thus separated by precipitation, and is recrystallized from isopropanol (2.5 g).

The methanol solution is evaporated and distilled at low pressure (3 mmHg): the 2,4,6-octatrienal separates at 55°-60°C and is purified on a silica gel flash chromatography column, eluting with a mixture of petroleum ether/ethyl acetate (ratio 98:2). 5 g of 2,4,6-octatrienal is obtained.
TLC: Hexane 7/Acetone 3

### Example 1B - Preparation of aldehydes with an even number of double bonds

Formula (**I**), n = 2: 2,4-hexadienal
n = 4: 2,4,6,8-decatetraenal
n = 6: 2,4,6,8,10,12-tetradecahexaenal

190 ml of acetaldehyde and 140 ml of crotonaldehyde are placed in a 1-litre flask, and kept under magnetic stirring for 30 minutes under nitrogen flow. 2 ml of piperidine and 1.4 ml of acetic acid are slowly dropped therein, and the mixture is left under magnetic stirring in a nitrogen atmosphere for 18 hours. 500 ml of ethyl ether are added to the red solution; the precipitate is filtered, then recrystallized from toluene. The precipitate obtained is 2,4,6,8,10,12,14-hexadecaheptaenal (140 mg), whereas 2,4,6,8,10,12-tetradecahexaenal (25 mg) is isolated from the evaporated toluene phase.

The red ether solution is washed with 200 x 5 ml of distilled water, dried over sodium sulphate and left to stand at -20°C for 12 hours. 2,4,6,8,10,12-tetradecahexaenal thus separated by precipitation is filtered and washed with ether (50 mg).

The residue obtained from evaporation of the organic phase is subjected to fractional distillation: unreacted crotonaldehyde is obtained at 21°C (30 mmHg), 2,4-hexadienal at 26°C (3 mmHg, 5.2 g) and 2,4,6-octatrienal at 55-60°C (3 mmHg). The residue is taken up with 30 ml of 80% methanol; 2,4,6,8-decatetraenal is separated by precipitation at -20°C and then filtered and recrystallized from hexane (420 mg).

The fraction of distillate containing 2,4,6-octatrienal is purified on a silica gel flash chromatography column, eluting with a mixture of petroleum ether/ethyl acetate (ratio 98:2), to obtain 3 g of 2,4,6-octatrienal.
TLC: Hexane 7/Acetone 3.

### Example 2 - Preparation of 2,4,6,8,10,12-tetradecahexaenol

2.2 mmols of 2,4,6,8,10,12-tetradecahexaenal are dissolved in 20 ml of anhydrous ethanol in a flask under nitrogen flow; 3.3 mmols of NaBH_{4,} solubilized in 5 ml of anhydrous ethanol, are dropped into the solution. The mixture is left at room temperature under magnetic stirring in a nitrogen atmosphere, checking periodically with TLC (ethyl acetate). After reacting for 5 hours, 30 ml of distilled water are added and the mixture is extracted with diethyl ether; the combined organic phases are dried over anhydrous sodium sulphate and evaporated under pressure (30 mmHg). The resulting crude product is purified by crystallisation from ethyl acetate. 2,4,6,8,10,12-tetradecahexaenol is obtained with a 60% yield.

The alcohols described in the table below were prepared in accordance with the procedure described above.

| No. | NAME | Reaction time | Purification | % yield |
|---|---|---|---|---|
| 2 | 2,4-hexadienol ⁽¹⁹⁾ | 1 hour | | 84 |
| 3 | 2,4,6-octatienol ⁽¹⁹⁾ | 1½ hours | Recrystallized from petroleum ether | 70 |
| 4 | 2,4,6,8-decatetraenol ⁽¹⁹⁾ | 2 hours | Recrystallized from ethanol | 70 |
| 5 | 2,4,6,8,10-dodecapentaenol ^{(20, 21)} | 3 hours | Recrystallized from ethyl acetate | 78 |
| 6 | 2,4,6,8,10,12,14-hexadecaheptaenol | 5 hours | Chromatography column, eluting with ethyl acetate | 40 |

### Example 3 - Preparation of 2,4,6-octatrienyl palmitate

2 mmols of 2,4,6-octatrienol under nitrogen stream are solubilized in anhydrous CHCl₃ not stabilized with ethanol; 2 mmols of anhydrous triethylamine are dropped into the solution. Keeping the flask under magnetic stirring in an ice bath, 3 mmols of palmitoyl chloride are slowly dropped into 10 ml of CHCl₃. When the addition is finished, the reaction mixture is brought to room temperature, checking periodically with TLC (ethyl acetate). The reaction is complete after approx. 5 hours. The reaction solution is extracted with distilled water and then with a saturated aqueous solution of NaHCO₃ dried over anhydrous sodium sulphate and evaporated (30 mmHg).

2,4,6-Octatrienyl palmitate with the following spectroscopic characteristics is obtained (yield 91 %):
- IR (KBr):: 2920, 2850, 1740, 1560, 1264, 996.
- ¹H-NMR (CDCl₃):: ppm 6.372-6.040 (4H, m, H₃₋₆); 5.791-5.959 (2H, m, H₂, H₇); 4.634 (2H, d, J_{1,2}= 5.64, CH₂O); 2.326 (2H, t, J_{5,6} = 7.3, CH₂CO); 1.816 (3H, d, J_{8,7} = 7.3, CH₃-CH=); 1.430-1.157 (26H,m, CH₂); 0.928 (3H, t, J = 6.23, CH₃).
- ¹³C (CDCl₃):: ppm 173.648 COO; 134.687 C₄; 134.454 C₃; 134.454 C₂; 134.454 C₅; 134.454 C₆; 134.454 C₇; 64,709 CH₂O; 34.430-22.738 CH₂; 18.309 CH₃-CH=; 14.134 CH₃.

The esters described in the table below were prepared in accordance with the procedure described above.

| No. | NAME | Reaction time | % yield |
|---|---|---|---|
| 2 | 2,4-hexadienyl palmitate | 4 hours | 94 |
| 3 | 2,4,6,8-decatetraenyl palmitate | 24 hours | 86 |
| 4 | 2,4,6,8,10-dodecapentaenyl palmitate | 24 hours | 84 |
| 5 | 2,4,6,8,10,12-tetradecahexaenyl palmitate | 24 hours | 60 |
| 6 | 2,4,6,8,10,12,14-hexadecaheptaenyl palmitate | 24 hours | 40 |

### Example 4 - Preparation of inclusions of aldehydes in cyclodextrins

Equimolar quantities of polyene aldehyde and α or β-cyclodextrin are mixed and worked in a mortar until a homogenous mixture is obtained. The mixture is worked for 30 minutes, added with 2 ml of distilled water in two successive times; the semi-liquid mixture thus obtained is placed into a flask kept in a nitrogen atmosphere overnight, and resuspend in 200 ml of warm distilled water (40°C). The coloured suspension is kept under magnetic stirring at 40°C for 20 minutes, then filtered while hot under vacuum (30 mmHg) through a sintered glass filter. A small amount of the yellow solution, stored at 4°C, does not contain any sediment or precipitate, even after several days. The product to be characterized is obtained by evaporation under vacuum (30 mmHg) of the aqueous solution.

Inclusions of polyene aldehydes containing 3, 4, 5, 6 and 7 conjugated double bonds in α and β-cyclodextrin were prepared in accordance with this procedure. Each complex obtained was characterized by IR, Raman and UV/vis spectroscopy.

**2,4,6-octatrienal included in α-cyclodextrin**

| | |
|---|---|
| IR (cm⁻¹) | 2929; 1680; 1641; 1413; 1384; 1298, 1244, 1155; 1078; 1030; 951. |
| Raman (cm⁻¹) | 1633, 1611; 1160; 1129. |
| UV/vis | in H₂O: λ (nm): 277.5 ε (1 cm⁻¹ mol⁻¹):705 |

**2,4,6-octatrienal included in β-cyclodextrin**

| | |
|---|---|
| IR (cm⁻¹) | 2927; 1667; 1644; 1417; 1369; 1302; 1247; 1158; 1080; 1028; 947. |
| Raman (cm⁻¹) | 1678; 1636; 1126. |
| UV/vis | in H₂O: λ (nm): 276.5 ε (1 cm⁻¹ mol⁻¹): 604 |

Biochemical and pharmacological experiments conducted on the compounds according to the invention have led to a characterization which indicates that they have a potential role in the prevention and treatment of various common disorders.

Their activity against the lipoperoxidation induced by CCl₄ on isolated rat hepatocytes, and against the oxidative phenomena induced by H₂O₂ on the phaeochromocytoma cell (PG 12), has been demonstrated by *in vivo* and *in vitro* tests.

Their ability to prevent hydroperoxide damage has also been demonstrated in a suspension of red blood cells placed in contact with hydrogen peroxide.

This test demonstrated that the compounds according to the invention have an elective inhibitory capacity against the damage caused by the toxic action of hydroperoxides.

Inhibition of collagen-induced platelet aggregation, and even more significantly, a reduction in reperfusion damage in the heart of the rat, has been found in the experimental cardiovascular field.

### Protection against H₂O₂-induced oxidation

A culture of phaeochromocytoma cells (PC-12) containing 3 x 10⁻⁵ M cells/ml was used; according to the method described by Nordman (Nordman R., Free Rad. Biol. Med., 1227, 1996) it was subjected to H₂O₂ at the concentration of 0.1 mM for 30 min.. 100 or 200 mcg/cc of "parrodin" (ie. the mixture of aldehydes with formula (**I**), wherein R = CHO) was added to the cell culture at the start of the experiment.

After 24 hours' incubation it was observed that with the peroxidation induced by H₂O₂ the survival rate was 30%, whereas the survival rate of the cells incubated with parrodin as well as H₂O₂ was 50% at the concentration of 100 mcg/cc and 90% at the concentration of 200 mcg/cc, thus demonstrating that parrodin significantly protects against the peroxidative damage caused by H₂O₂.

### Tests of lipoperoxidation induced by CCl₄.

The lipoperoxidative and toxic effect induced by CCl₄ (100 mg/L⁻¹) was evaluated on rat hepatocytes isolated according to the technique described by Segler (Segler P.O., Methods Cell. Biol. Chem., 264, 4747, 1989). CCl₄ is known to cause lipoperoxidation of the cell membranes which can lead to cell necrosis (Slater T.F., Philos Trans. R. Soc. Lond. (Biol) 311, 633, 1985; Berger M.L., Hepatology, 6, 36, 1996; Tribble D.L., Hepatology, 7, 377, 1987).

The damage to the cell membrane caused by CCl₄ and the protective effect performed were measured by assaying alanine aminotransferase (AlaAT) and aspartate aminotransferase (AspoAT) on the supernatant liquid of the cell culture (Auto-biochemistry Assay System-Beeckman 700-Encore-2).

Cytological tests on the hepatocytes were performed under the optical or electronic microscope after they had been fixed in formalin or glutaraldehyde. The results of these tests demonstrated that the increase in AlaAT and AspAT concentrations caused by CCl₄ is reduced by the presence of parrodin.

The histological tests also demonstrated the protection provided by parrodin. The cell membranes and nucleus of the treated hepatocytes, unlike the controls, appeared almost intact, and the mitochondria and the number of ribosomes also appeared normal.

### Test of lipoperoxidation induced by hydrogen peroxide on red blood cells

Red blood cells were extracted from the venous blood of healthy volunteers; after centrifugation and washing in saline buffered with PBS phosphates (0.15M, pH 7.4) they were diluted with 10 cc of a solution containing 10⁻³M of PBS-azide, and the haemoglobin concentration was measured with Drabkin's reagent. Lipid peroxidation was induced by exposing a cell expansion contained in 5 cc of PBS-azide with a final haemoglobin concentration of 3.75 mg/ml to hydrogen peroxide (20 mM of hydrogen peroxide per ampoule containing 5 cc of cell suspension) and incubating them at 37° for one hour.

Peroxidation was determined after one hour according to the Stocks and Dormandy method (Stocks J., Dormandy T.L., Brit. J. Haematology, 29, 95, 1971) that measures the formation of malonaldehyde which, in combination with thiobarbituric acid (TBA), forms a coloured chromogen with absorbance at 532 nm (Bird R.P., Methods Enzymol, 105, 299, 1984).

The antilipoperoxidative activity of parrodin was evaluated by introducing it into tubes containing the erythrocyte suspension at the dose of 100 mcg/ml.

The MDA measurement after one hour's incubation with hydrogen peroxide demonstrates a highly significant reduction of MDA formation by parrodin, and consequently evident protection against lipoperoxidation damage.

### Platelet aggregation tests

Platelet aggregation was determined on platelet-rich plasma (PRP); the number of platelets was counted with a CH58 oH platelet counter (Delcan) and made up to 300,000 platelets/ml with platelet-poor plasma (PPP).

Platelet aggregation was induced by adding collagen (2.5 ng/ml) and evaluated photometrically with an aggregometer according to the technique described by Born (Born G.V.R., Nature 194, 927, 1962).

After 10 minutes' incubation with Parrodin (2.5 ng/ml and 5 mg/ml), 55% and 75% inhibition of platelet aggregation respectively was obtained.

### UV-induced erythema prevention tests

UV-induced erythema was caused by applying a UV lamp (Hanoivna Kramager) to the ear of a guinea pig for 30 sec; the lamp transmitted rays with a wavelength of 200 to 400 mm, which consequently included UV-B, UV-C and UV-A rays, through a special filter.

Excipients only or a suspension containing 5 or 10% 2,4,6,8,10,12,14-hexadecaheptaenol palmitate were spread on the ear of the test animals, and the temperature increase resulting from the UV-induced erythema was measured with a thermoelectric thermometer after approx. 3 hours in the control animals and the test animals. It was found that the 10% suspension inhibited the appearance of heat and erythema.

The set of tests performed indicates that the polyunsaturated linear compounds according to the invention possess the biological characteristics common to carotenoids of plant or other origin, and demonstrate a particular protective activity against hydroperoxides and free radicals.

They consequently seem likely to be particularly useful in the prevention and treatment of all the organic changes caused by the activation of free radical production. They can be used as diet supplements, medicaments or cosmetics in the prevention or treatment of various disorders associated with tissue aging. In cosmetology in particular, they can be used to treat lesions caused by ultraviolet rays and skin aging processes, inflammatory or degenerative reactions.

Depending on their use, the compounds according to the invention can be employed alone or in association with one another, or in association with other carotenoids or compounds with a similar, complementary activity, such as organic or inorganic antioxidants, vitamins, aminoacids, enzymes or other products with nutritional characteristics or characteristics available in cosmetology.

The compounds according to the invention may be formulated in the form of ointments, creams or lotions for topical cosmetic cutaneous use.

The formulations will be prepared according to conventional techniques, using excipients suitable for pharmaceutical or cosmetological use.

Some examples of formulations according to the invention are set out below.

### Formulation example 1

### For topical use

(cream, ointment, lotions) - content/cc
Parrodienes
5% cyclodextrin -
dodecapentaenal hexadecapentaenal complex

### Formulation example 2

### Complexes based on Parrodienes for oral use

| | |
|---|---|
| "Parrodin" cyclodextrin complex | 5 mg |
| Lycopene | 5 mg |
| β-carotene | 1 mg |
| Vit. E | 5 mg |
| Vit. C | 50 mg |
| Coenzyme Q₁₀ | 20 mg |
| Selenium | 50 mcg |
| "Parrodin" cyclodextrin complex | 5 mg |
| Alpha-lipoic acid | 50 mg |
| Coenzyme Q₁₀ | 30 mg |
| Vit. C | 50 mg |
| Vit. E | 5 mg |
| Vit. B₁ | 5 mg |

### Formulation example 3

### Complex based on Parrodienes for cosmetic use

| | |
|---|---|
| Cream containing the following in each cc "Parrodin" cyclodextrin complex | 5 mg |
| Melatonin | 10 mg |
| Alpha-lipoic acid | 50 mg |
| Vit. E | 10 mg |

### REFERENCES

1) Brown J.E.,Clin.Chim.Acta,193,147, 1990
2) Haglund O.,J.Int.Med.,227,347, 1990
3) Parks J.S.,Atherosclerosis,84,83, 1990
4) Gross D.,Therapie Woche,33,4238, 1984
5) Thico G.,Schweiz.Rundschau Med. (Praxis),66,1696, 1977
6) Vaz A.L.,Curr.Med.Res.Opin.,8,145, 1982
7) Schwarz K.,J.Biol.Chem.,265,22023, 1990
8) Theodosakis J.,The Arthritis Cure St.Martin's Press. N.Y., 1997
9) Ferenc Levay P.,Haematologica,80,252, 1995
10) Playford R.J.,Am.J.Nutr.,72,5, 2000
11) Whitheouse M.W,Inflam.pharmacol.,5,237, 1997
12) Rainsford K.D.,Arzneimitt.Forschung,30,2128,1980
13) Isler O.,Carotenoids,Birkhauser Verlag Basel, 1971
14) Goodwin T.W.,The Biochemistry of carotenoids in plants, vol.1 - Chapman Hall London 1980
15) Goodwin T.W.,The Biochemistry of Carotenoids, vol.2 Animals - Chapman Hall London 1984
16) Krinsky N.Ann.Rev.Nutr.,13,561, 1993
17) Kuhn, R., and Grundmann, Chem.Ber., 70, 1318 (1937)
18) Blout, E.R, Fields, M., J.A.C.S., 70, 189-193 (1948)
19) Reichstein; Ammann; Trivelli, Helv. Chim. Acta 15, 263-267 (1937)
20) Fischer; Hultzsch; Flaig, Chem. Ber. 70, 374 (1937)
21) Synder, Richard; Anridson, Eric; Foote, Caroline; Harrigan, Lynne; Christensen, Ronald L J.Amer.Chem.Soc. 107,14, 4117 (1985)

## Claims

1. A cosmetic method of treatment of skin damage caused by ultraviolet rays and ageing processes which comprises the topical application of compounds with the general formula (**I**): 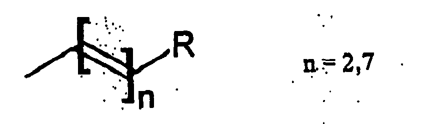 wherein
n = 2 - 7,
R = CHO, CH₂OH, CH₂O-C-(O)R', wherein -CO-R' is the residue of a fatty acid with 12-22 carbon atoms,
alone or in a mixture thereof.

2. As a novel compound, 2,4,6,8,10,12-tetradecahexaenol of formula (**I**), wherein n = 6 and R = CH₂OH.

3. As a novel compound, 2,4,6,8,10,12,14-hexadecaheptaenol of formula (I), wherein n = 7 and R = CH₂OH.

4. As novel compounds, the compounds of general formula (**I**), wherein n = 2, 3, 4, 5 or 6 and R = CH₂O-CO-R', wherein -CO-R' is the residue of palmitic acid.

5. Inclusion compounds of compounds with formula (**I**) in cyclodextrins 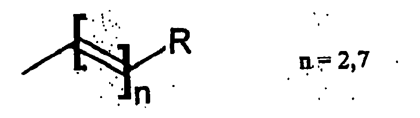 wherein
n = 2-7 and R = CHO.

6. The use of compounds of formula (I) 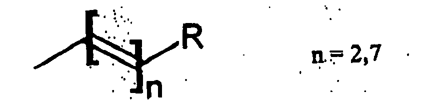 for the preparation of a topical composition having anti-oxidant and anti-inflammatory activity.

7. The use of compounds of formula (I) 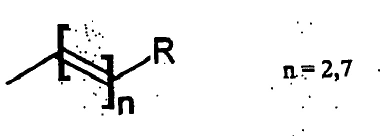 for the preparation of a topical composition for the treatment of skin damage caused by ultraviolet rays and ageing processes.

## Patentansprüche

1. Verwendung von Verbindungen der allgemeinen Formel (I) : 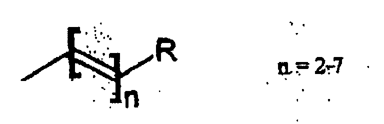 worin
n = 2 - 7,
R = CHO, CH₂OH, CH₂O-C-(O)R', worin -CO-R' für den Rest einer Fettsäure mit 12-22 Kohlenstoffatomen steht,
alleine oder in einem Gemisch davon topisch aufträgt, für die Herstellung einer topischen Zusammensetzung zur Behandlung von durch ultraviolette Strahlen und Alterungsprozesse verursachten Hautschäden.

2. 2,4,6,8,10,12-Tetradecahexaenol der Formel (I), worin n = 6 und R = CH₂OH.

3. 2,4,6,8,10,12,14-Hexadecaheptaenol der Formel (I), worin n = 7 und R = CH₂OH.

4. Verbindungen der allgemeinen Formel (I), worin n = 2, 3, 4, 5 oder 6 und R = CH₂O-CO-R', worin -CO-R' für den Rest von Palmitinsäure steht.

5. Einschlußverbindungen von Verbindungen der Formel (I) in Cyclodextrinen 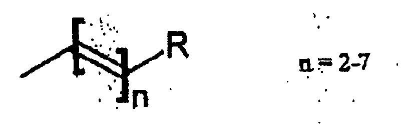 worin
n = 2 - 7 und R = CHO.

6. Verwendung von Verbindungen der Formel (I) 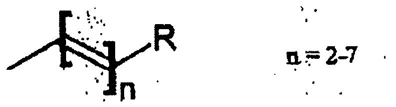 für die Herstellung einer topischen Zusammensetzung, die eine Antioxidations- und entzündungshemmende Aktvität hat.

## Revendications

1. Utilisation de composés de formule générale (I) : 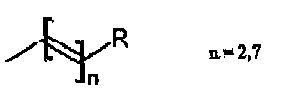 dans laquelle
n = 2-7,
R = CHO, CH₂OH, CH₂O-C-(O)R', où -CO-R' est le résidu d'un acide gras avec 12-22 atomes de carbone,
seuls ou dans un mélange de ceux-ci, pour la préparation d'une composition topique pour le traitement de lésions de la peau causé par des rayons ultraviolets et des processus de vieillissement.

2. 2,4,6,8,10,12-tétradécahexaénol de formule (I), dans laquelle n = 6 et R = CH₂OH.

3. 2,4,6,8,10,12,14-hexadécaheptaénol de formule (I), dans laquelle n = 7 et R = CH₂OH.

4. Composés de formule générale (I), dans laquelle n = 2, 3, 4, 5 ou 6 et R = CH₂O-CO-R', où -CO-R' est le résidu de l'acide palmitique.

5. Composés d'inclusion de composés de formule (I) dans des cyclodextrines 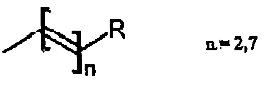 dans laquelle
n = 2-7 et R = CHO.

6. Utilisation de composés de formule (I) 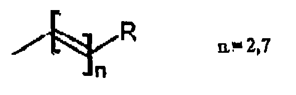 pour la préparation d'une composition topique ayant une activité antioxydante et anti-inflammatoire.
